# EUROPÄISCHE PATENTANMELDUNG

(11) **EP 1 510 181 A1**
(43) Veröffentlichungstag der Anmeldung: **02.03.2005**
(21) Anmeldenummer: 03019315.5
(22) Anmeldetag: 27.08.2003
(51) Int. Cl.: A61B 19/00

(54) **Verwendung eines passiven Zeigeinstrumentes als interaktives Eingabemedium**

(71) Anmelder: CAS Innovations AG, 91056 Erlangen (DE)
(72) Erfinder: Huwer, Stefan, 91056 Erlangen (DE)

(57) **Zusammenfassung**

Es wird eine Erfindung vorgestellt, deren Anwendungsbereich sich auf die Unterstützung von chirurgischen Eingriffen mittels Navigationsstationen erstreckt. Bei der Erfindung handelt es sich um eine Kombination eines passiven Zeigeinstrumentes zur navigierten Positionsbestimmung mit einer speziellen Software als Teil einer Navigationsstation, deren Ziel es ist die interaktive Kontrolle der Punktaufnahme über die Detektion einer charakteristischen Bewegung des passiven Zeigeinstrumentes zu realisieren. Durch die Erweiterung der Navigationsstation um die Möglichkeit mit Hilfe eines passiven Zeigeinstrumentes eine Punktaufnahme zu initiieren wird eine deutliche Verbesserung der Handhabbarkeit des Systems erzielt. Die Navigationsstation kann nun während der Positionsaufnahme alleine vom Mediziner, der mit dem Zeigeinstrument arbeitet, bedient werden ohne dass er dabei seine Aufmerksamkeit von der Operationsfläche abwenden muss.

## Beschreibung

### 1. Beschreibung

Bei der beschriebenen Erfindung handelt es sich um eine Kombination eines passiven Zeigeinstruments zur navigierten Positionsbestimmung mit einer speziellen Software als Teil einer Navigationsstation zur Unterstützung von chirurgischen Eingriffen. Die vorgestellte Erfindung bezieht sich auf eine Navigationsstation unter Verwendung passiver Zeigeinstrumente, welche keine aktiven Signale aussenden.

### Problemstellung und Stand der Technik

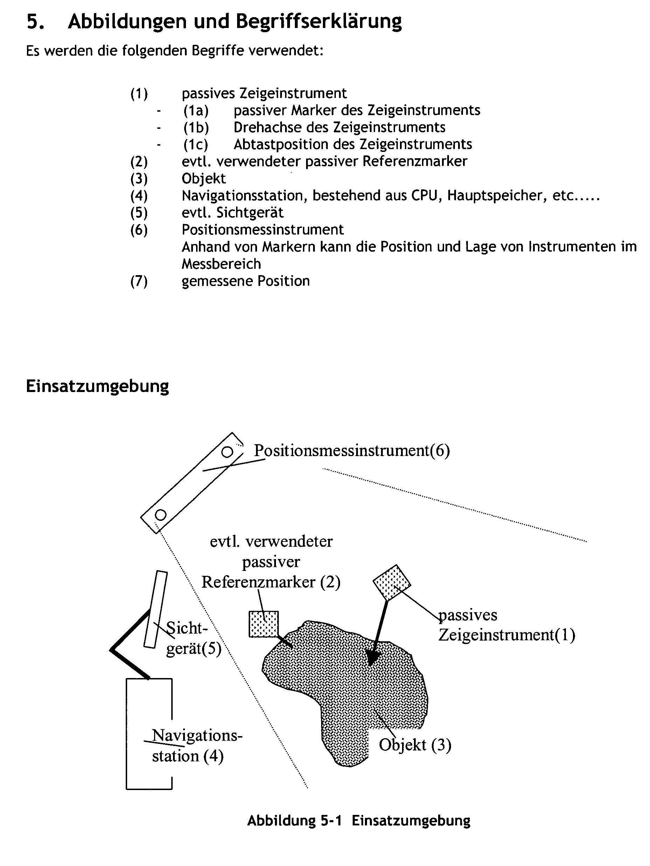

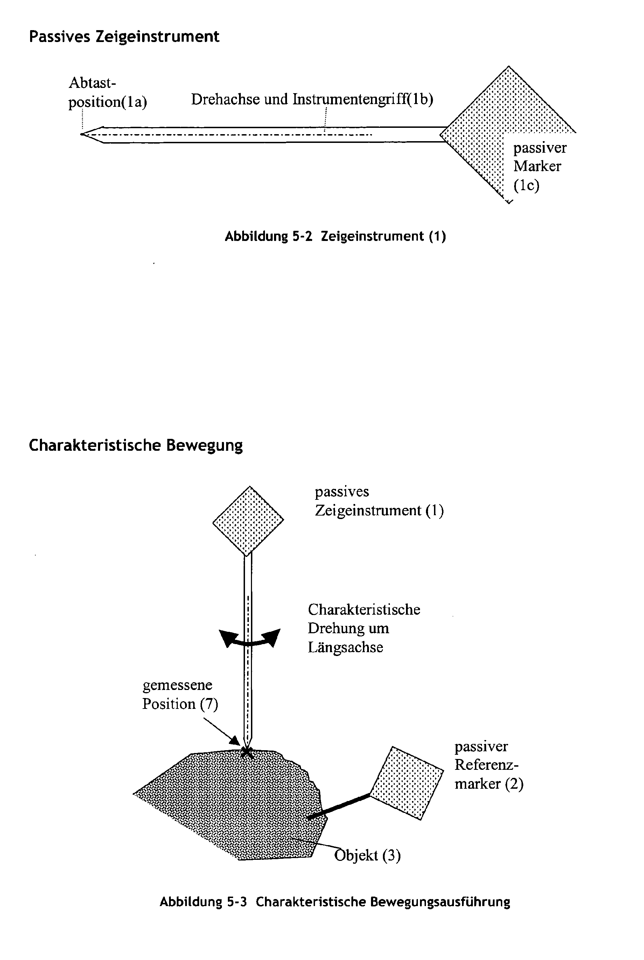

Chirurgische Eingriffe werden vermehrt navigiert durchgeführt. Zum Einsatz kommt dabei operationsunterstützend eine Navigationsstation (siehe in Abbildung 5-1), bestehend aus einem Rechner, einem Sichtgerät (meist Monitor), einem Eingabemedium (meist Tastatur oder Touchscreen), und einem Messinstrument zur schritthaltenden Aufnahme der Instrumenten- und Patientenpositionen im 3D (meist eine Stereokamera). Über den Rechner werden mittels spezieller Analyseverfahren die Informationen des Messinstruments in eine für den Anwender unterstützenden Art und Weise aufbereitet. Es können dabei Navigationsstationen auf Basis aktiver Marker und passiver Marker, unterschieden werden.

Während eines navigiert durchgeführten chirurgischen Eingriffs ist die folgende Problemstellung bekannt: Durch das Anfahren von anatomischen Punkten am oder im Patienten werden analytische Auswertungen durch die Navigationsstation ermöglicht. Bei der Aufnahme von anatomischen Punkten können dabei sowohl Einzelmessungen von Instrumentpositionen, sowie ganze Messreihen von Instrumentpositionen durchgeführt werden. Um die Messung durchzuführen oder eine Messreihe zu starten, werden nach dem aktuellen Stand der Technik dem Anwender Bedienelemente, wie z.B. Tastatur, Touchscreen oder Fußschalter, etc. über die Navigationsstation zur Verfügung gestellt. Über diese Bedienelemente erfolgt die Interaktion mit dem Navigationssystem.

### Nachteile dieser Interaktionsart

Die Nachteile einer Interaktion des Anwenders mit der Navigationsstation bestehen in der Ablenkung der Aufmerksamkeit des Anwenders von der Aufgabe korrekte anatomische Punkte abzutasten, auf die Bedienung eines Eingabemediums.

Weiterhin sind Nachteile in der eventuell nicht möglichen Einpersonenbedienbarkeit des Systems zu sehen, da der Anwender gleichzeitig das Eingabemedium sowie das Zeigeinstrument bedienen muss. Räumliche Verhältnisse können diese gleichzeitige Bedienung durch einen Anwender unmöglich machen.

### 2. Ziele

Ziel dieser Erfindung ist die Bereitstellung eines Verfahrens, das die interaktive Kontrolle der Punktaufnahme über ein passives Zeigeinstrument, direkt über die Ausführung einer charakteristischen Bewegung des Instrumentes bereitstellt und somit die oben genannten Nachteile des Standes der Technik überwindet. Die Kontrolle der Positionsaufnahme durch den Anwender wird so von den üblichen Eingabemedien der Navigationsstation (Tastatur, Touchscreen) zum passiven Zeigeinstrument verlagert. Durch die Erweiterung der Navigationsstation um die Möglichkeit mit Hilfe eines passiven Zeigeinstrumentes eine Punktaufnahme zu initiieren wird eine deutliche Verbesserung der Handhabbarkeit des Systems erzielt. Die Navigationsstation kann nun während der Positionsaufnahme alleine vom Mediziner, der mit dem Zeigeinstrument arbeitet, bedient werden ohne seine Aufmerksamkeit von der Operationsfläche abwenden zu müssen.

### 3. Lösung

Der über die Patentansprüche angegebenen Erfindung liegt die Aufgabe zugrunde die direkte Integration der Eingabemöglichkeit in das passive Instrument zu ermöglichen, indem die Navigationsstation mit einem Verfahren zur Erkennung einer charakteristischen Bewegung des Zeigeinstrumentes ausgestattet wird. Nach der Erkennung dieser charakteristischen Bewegung wird eine Positionsmessung durchgeführt. Diese Positionsmessung kann einerseits eine Einzelpunktmessung oder aber auch die Messung einer gesamten Positionsmessreihe sein. Wird während der Aufnahme einer Messreihe erneut die charakteristische Bewegung detektiert, wird die Messreihe beendet.
Ein Ausführungsbeispiel eines für diese Erfindung verwendbaren Zeigeinstrumentes ist in Abbildung 5-2 dargestellt.
Die Aufgabe ist erfindungsgemäß gelöst, durch die Merkmale des Patentanspruchs. Die Erfindung ist nachfolgend erläutert.

### Charakteristische Bewegung

Die charakteristische Bewegung besteht in einer Drehung um die Längsachse des Zeigeinstrumentes, bei nahezu unbewegter Spitze des Zeigeinstruments (siehe Abbildung 5-3 ) innerhalb eines gegebenen Detektionszeitraums fester Länge. Die Unbewegtheit der Spitze des Zeigeinstruments kann hierbei in Relation zu einem Referenzmarker entschieden werden. Die Bedingungen der charakteristischen Bewegung lassen sich somit folgendermaßen beschreiben:
Das Zeigeinstrument erfüllt innerhalb eines gegebenen Detektionszeitraumes, die folgenden beiden Eigenschaften:
   - Es wurde eine Drehung um eine Instrumentenlängsachse von mindestens r Winkelgraden durchgeführt.
   - Die Zeigeinstrumentenspitze weisst eine Positionsschwankung von maximal d Längeneinheiten auf.

Zusammenfassend kann die charakteristische Bewegung, welche zum Auslösen einer Positionsaufnahme oder Messreihenaufnahme notwendig ist anhand von drei Parametern (Detektionszeitraum, Mindestrotation, Maximalpositionsschwankung) festlegen.

### Erkennung der charakteristischen Bewegung

Die Navigationsstation wird mit einem Verfahren zur schritthaltenden Analyse der Bewegungsdynamik eines Zeigeinstruments zur Detektion der charakteristischen Bewegung ausgestattet. Hierzu werden schritthaltend die Positionen des Zeigeinstrumentes ausgewertet. Erfüllen die Positionen über einen gegebenen Detektionszeitraum die oben genannten Bedingungen der charakteristischen Bewegung, dann wird eine charakteristische Bewegung erkannt.

### Lösung ohne Referenzmarker

Die Navigationsstation liefert schritthaltend die Lagen (bestehend aus Position und Rotation) des Zeigeinstruments im durch die Navigationsstation bestimmten Koordinatensystem. In diesem Koordinatensystem werden die Bedingungen der charakteristischen Bewegung überprüft.

### Lösung mit Referenzmarker

Die Navigationsstation liefert schritthaltend Positionen des Referenzmarkers sowie des Zeigeinstruments im durch den Referenzmarker bestimmten Koordinatensystem. Die Bedingungen der charakteristischen Bewegungen werden im Koordinatensystem des Referenzmarkers überprüft, so dass die Bewegungen des Referenzmarkers bereits berücksichtigt sind.

### Verfahren

Mit Methoden der linearen Algebra werden zu den gegebenen Positionen des Zeigeinstrumentes, sowie dessen Orientierung, die Bedingungen der charakteristischen Bewegung überprüft.
Die Bestimmung der maximalen Abweichung der Zeigeinstrumentenspitze während des Beobachtungszeitraums lässt die Prüfung der Bedingung 2 zu. Die Bedingung 1 wird geprüft, indem eine zur Längsachse des Zeigeninstruments senkrechte Achse betrachtet wird. Während des Beobachtungszeitraums wird nun geprüft, dass der maximal auftretende Winkel zwischen zwei Achsenrichtungen größer als die geforderte Mindestrotation wird, wobei die Längsachse des Zeigeinstruments nahezu unbewegt bleibt.

## Patentansprüche

1. Verfahren zur interaktiven Steuerung einer Navigationsstation anhand der schritthaltenden Analyse des Bewegungsmusters eines passiven Zeigeinstrumentes.

2. Kombination der Auswertung der charakteristischen Bewegung eines Zeigeinstrumentes, bestehend aus einer Drehung um die Längsachse bei fix gehaltener Spitze, mit einer Navigationsstation zur Steuerung interaktiver Prozesse.

3. Verfahren zur Aufnahme einer Position eines Zeigeinstrumentes anhand der Durchführung der charakteristischen Bewegung mit dem passiven Zeigeinstrument.

4. Verfahren zum Starten einer Messreihe von Punktaufnahme anhand der Durchführung der charakteristischen Bewegung des passiven Zeigeinstrumentes.

5. Verfahren zum Beenden einer Messreihe von Punktaufnahmen anhand der Durchführung der charakteristischen Bewegung des passiven Zeigeinstrumentes.
